# DEMANDE DE BREVET EUROPEEN

(11) **EP 0 976 371 A1**
(43) Date de publication de la demande: **02.02.2000**
(21) Numéro de dépôt: 98401955.4
(22) Date de dépôt: 30.07.1998
(51) Int. Cl.: A61F 2/80

(54) **Technique de fabrication pour manchon gel uréthanne**

(71) Demandeur: Garnier, philippe, 75019 Paris (FR)
(72) Inventeur: Garnier, philippe, 75019 Paris (FR)

(57) **Abrégé**

Manchon en gel d'uréthanne destiné aux amputés pour servir d'interface entre la peau et l'emboîture d'une prothèse.

L'invention consiste en une technique de fabrication des manchons en gel d'uréthanne à partir de plaque de ce même gel, ce qui permet de se dégager d'une réalisation par système de moule et contre moule.

Le contour de la surface développée du moulage est découpé sur la plaque et assemblé bord à bord par collage.

La forme ainsi obtenue, est enduite du mélange résine et catalyseur pour coller sur le manchon intérieurement et extérieurement le tissu textile.

La technique de fabrication selon l'invention est particulièrement destinée à la réalisation de manchons sur mesures en gel d'uréthanne utilisés en orthopédie

## Description

La présente invention concerne une technique de fabrication de manchons en gel d'uréthanne et utilisée en orthopédie pour servir d'interface entre la peau et l'emboîture d'une prothèse permettant de suppléer à un membre amputé.

Actuellement la technique de fabrication de ces manchons de type standards ou sur mesure est la suivante :
- Réalisation d'un moule reproduisant la forme et les dimensions du moignon du patient.
- Réalisation d'un contre moule dont les dimensions intérieures sont de quelques millimètres supérieures à celles du moule.
- Dans l'espace annulaire entre moule et contre moule, est coulée une matière à base d'uréthanne. Après polymérisation, on enlève le moule et le contre moule et l'on obtient le manchon.

Si l'utilisation de cette technique se justifie pour la fabrication de manchons standards, par contre pour une fabrication sur mesure, elle est longue et coûteuse.

La présente invention concerne une technique de fabrication qui supprime les inconvénients cités plus haut.

Cette technique consiste à réaliser le manchon à partir d'une plaque d'uréthanne de dimensions et d'épaisseur appropriées. La première opération consiste à réaliser un moule en plâtre ou en un autre matériau appelé 〈〈 Positif 〉〉, à partir du moulage du moignon de l'amputé, reproduisant ainsi parfaitement sa morphologie.

La fabrication du manchon suivant cette technique s'effectue en plusieurs étapes successives :
a) Sur une plaque d'uréthanne de dimensions et d'épaisseur adéquates est reproduit le contour de la surface développée du 〈〈 positif 〉〉. Ce développé plan est obtenu par la mesure du contour du 〈〈 positif 〉〉 section par section.
b) Après découpe de la surface développée suivant le contour ainsi défini, les lèvres extérieures sont ensuite assemblées bord à bord sur toute la hauteur avec un filet de colle de 1mm déposé au milieu du chant d'une des lèvres. La forme ainsi créée peut s'emboîter parfaitement sur le 〈〈 positif 〉〉. La colle utilisée est une colle cyanoacrylate dont l'avantage est une prise instantanée, indispensable à la réalisation de formes complexes.
   Le collage peut aussi se faire avec des composants à base d'uréthanne de même formulation que ceux entrant dans la fabrication de la plaque, mais seulement pour des formes simples genre conique ou cylindrique. Dans ce cas, le collage des lèvres extérieures s'effectue suivant la technique du bord à bord, sur toute leur épaisseur en maintenant les bords en contact par un dispositif approprié pendant le temps nécessaire à la polymérisation.
c) Le positif est enduit d'un produit de démoulage à base de silicone et positionné sur un support permettant d'assurer une dépression.
d) Un mélange de résine polyuréthanne et d'un catalyseur est préparé puis dégazé sous cloche à vide.
e) Le 〈〈 positif 〉〉 est enduit de ce mélange sur toute sa surface puis recouvert d'un fin bas textile, par exemple en polyamide que l'on prendra soin de ne pas tendre exagérément pour conserver au gel d'uréthanne toutes ses propriétés physiques et mécaniques.
f) La forme obtenue à partir de la plaque d'uréthanne est enfoncée par dessus le bas textile sur le positif.
g) La forme précédemment citée est à son tour enduite du même mélange sur toute sa surface et recouverte d'un autre bas textile.
h) L'ensemble est ensuite recouvert d'un manchon standard en silicone qui va fermer hermétiquement l'ensemble sur le support à dépression.
i) Le vide est fait à 0,4 bar sous le support et l'ensemble est maintenu sous vide le temps nécessaire à la polymérisation du mélange.
On obtient suivant cette technique un manchon s'adaptant parfaitement au 〈〈 positif 〉〉 et donc à la morphologie du moignon du patient.
Cette innovation technique apporte un progrès important dans la réalisation de manchons en gel d'uréthanne sur mesure.

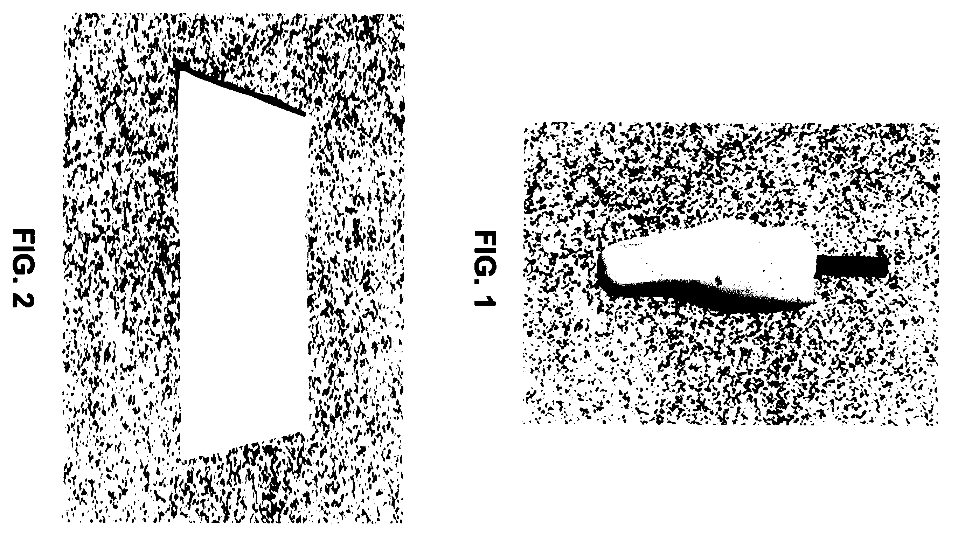

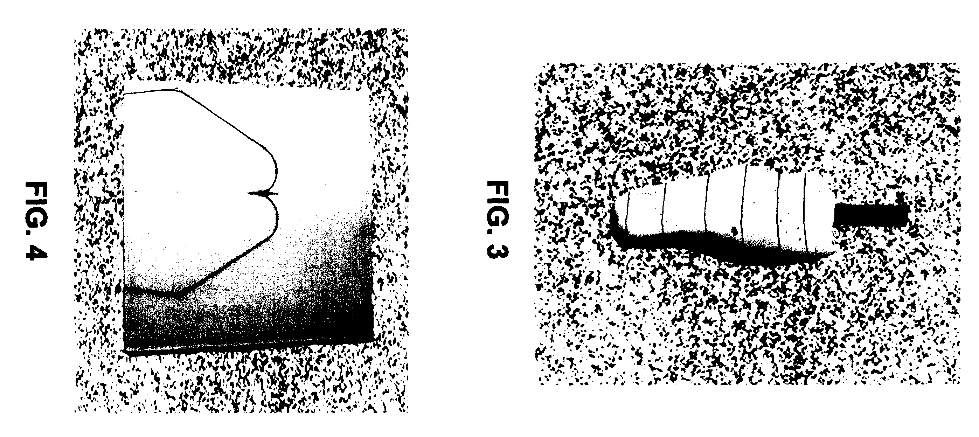

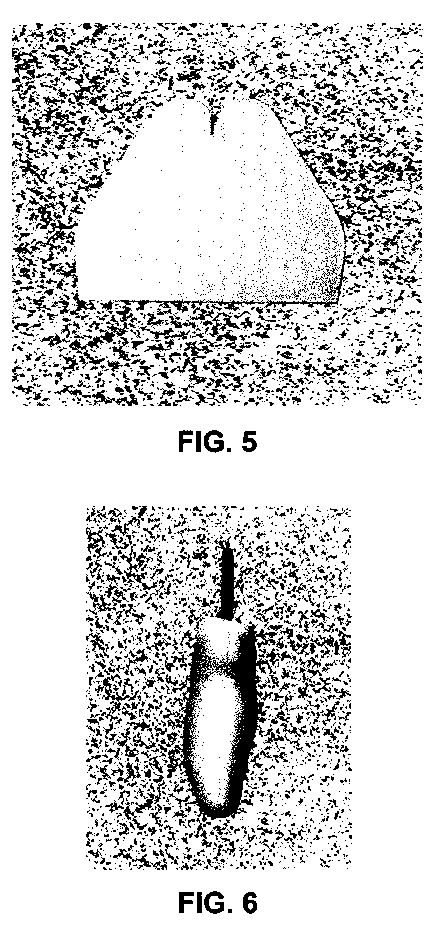

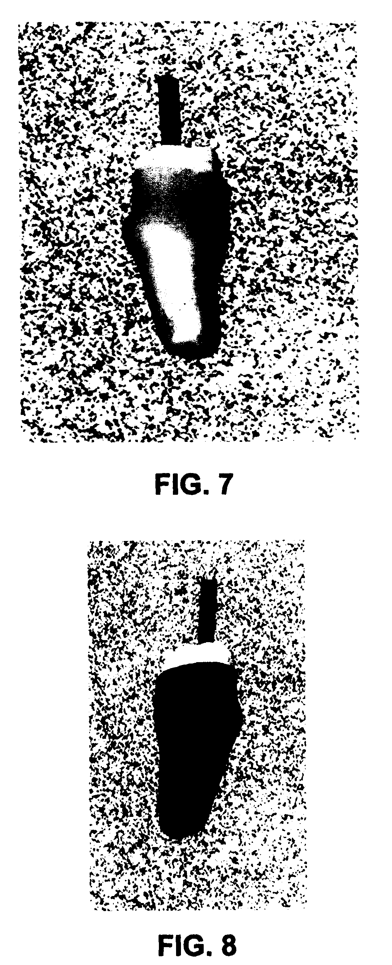

## Revendications

1. Fabrication de manchons en gel d'uréthanne, destinée à l'orthopédie caractérisés par (1) l'utilisation de plaques d'uréthanne, ainsi que (2) d'un système de collage avant (3) la réalisation.

2. Utilisation selon la revendication (1) de plaque d'uréthanne pour la fabrication du manchon.

3. Utilisation d'une technique de collage, selon la revendication (2) permettant de mettre en forme la ou les plaques de gel d'uréthanne.

4. Réalisation définitive du manchon selon la revendication (3) par une technique de sous vide utilisant un mélange de résine polyuréthanne et catalyseur pour lier ensemble le textile de finition et les plaques de gel.
